# EUROPEAN PATENT APPLICATION

(11) **EP 4 268 837 A1**
(43) Date of publication of application: **01.11.2023**
(21) Application number: 21911556.5
(22) Date of filing: 22.12.2021
(51) Int. Cl.: A61K 35/765, A61P 35/00

(54) **NOVEL MODIFIED REOVIRUS AND USE THEREOF**

(30) Priority: 22.12.2020 KR 20200180824
(71) Applicant: Virocure, Inc., Seoul 08381 (KR)
(72) Inventor: YOO, Haeng Jun, Seoul 05698 (KR); HAN, Sang Kyoung, Seoul 06703 (KR); LEE, Yeon Sook, Gunpo-si Gyeonggi-do 15821 (KR); SONG, Ki Hoon, Paju-si Gyeonggi-do 10879 (KR)
(74) Representative: Dehns
(86) International application number: PCT/KR2021/019663
(87) International publication number: WO 2022/139490

(57) **Abstract**

The present invention relates to a modified reovirus and use thereof, and has been completed by confirming that a novel modified reovirus derived from a wild-type reovirus not only has an excellent anticancer effect on various cancers, including rare cancers, but can also enhance the effect of cancer immunotherapy agents. Particularly, it was confirmed that the modified reovirus according to the present invention significantly reduced the cell viability of all cancer cell lines when used to treat various types of cancer cells, including rare cancers such as melanoma and tongue cancer, and in particular, had an excellent anticancer effect even on taxane-based anticancer drug-resistant cancer cell lines. Furthermore, the modified reovirus according to the present invention exhibited a dose-dependent anticancer effect even in melanoma, bladder cancer and oral cancer mouse models regardless of administration routes, and the anticancer effect was shown to be further increased upon repeated administration or alternate administration with a wild-type reovirus. In particular, it was confirmed that the modified reovirus according to the present invention exhibited a synergistic anticancer effect when used in combination with an immune checkpoint inhibitor. Therefore, the modified reovirus according to the present invention is expected to be effectively used as a new anticancer therapy for treating rare cancers and the like and as a drug used in combination with a cancer immunotherapy agent.

## Description

### [Technical Field]

The present invention relates to a new modified reovirus and its use.

This application claims priority based on Korean Patent Application No. 10-2020-0180824, filed on December 22, 2020, and Korean Patent Application No. 10-2021-0184947, filed on December 22, 2021, and all contents disclosed in the specification and drawings of the said applications are incorporated herein by reference.

### [Background Art]

Development of early diagnosis methods for cancer and continuous development of new anticancer therapies have improved the treatment efficacy of cancer. However, cancer remains a major cause of death worldwide. In 2015, 27.9% of the total deaths in South Korea (275,895 individuals) were due to cancer, making it the leading cause of death in the country. The incidence and mortality rates of cancer are continuously increasing.

Rare cancer is a cancer in which the prevalence population is less than 20,000 or exact prevalence is unknown because early diagnosis is difficult. They are defined according to the procedures and standards set by the Ministry of Health and Welfare in South Korea (Rare Disease Management Act, enforced on April 30, 2019). In South Korea, rare cancers, such as blood cancers, account for 16% of all cancer cases. However, most of the new drug development is focused on the top 10 types of cancer, which have a larger patient population. Therefore, there are very few cases of new drug development for rare cancers. For rare cancers, there is a problem of low treatment efficacy due to the lack of established guidelines and anticancer drugs for treatment, as well as difficulty in early diagnosis, leading to diagnosis at stage 3 or later, when metastasis has already taken place.

Representative rare cancers, such as tongue cancer and malignant melanoma, can cause skin ulcers and facial deformities during the treatment process, which can reduce the patient's quality of life and lead to complications such as depression or suicide in severe cases. Tongue cancer, a common type of oral cancer, occurs in the tongue within the oral cavity. Over 90% of oral cancers correspond to malignant tumors that originate from the squamous cells of the oral mucosa. Tongue cancer, a rare cancer accounting for less than 0.2% of all cancer patients, may result in sequelae such as decreased masticatory movement and orofacial deformities. If the appropriate treatment time is missed, tongue cancer, as a highly dangerous cancer, can result in a mortality rate of approximately 44% within five years after onset.

The incidence of oral cancer, including tongue cancer, in developing countries is as high as 13%. According to Mores et al., approximately 30,300 cases of oral cancer, including those of the tongue, mouth, pharynx, and other oral cancers, occurred in the United States in 1998, resulting in approximately 8,000 deaths (Oral Oncol. 1999 Jan;35(1):1-8). The occurrence trend of cancer varies, and an increasing trend has been observed in Eastern and Central Europe. Maria and Susan reported an incidence rate of 8.3 cases per 100,000 individuals for oral cancer until 1998, with over 95% of cases occurring in individuals aged 40 years or older. From 1975 to 1998, oral cancer occurred more frequently in males and had a higher incidence rate in the black population (Oral Oncol. 2002 Sep;38(6):610-7). In South Korea, it has been reported that approximately 3,000 cases of oral cancer, including tongue cancer, occur annually.

The etiology of melanoma is not fully understood, but it is known to result from a complex interplay of genetic and environmental factors, such as exposure to ultraviolet radiation. A family history of melanoma accounts for 10 to 15% of all cases, and the risk is doubled when there is a patient in the same generation. Among environmental factors, ultraviolet radiation, particularly ultraviolet B, is associated with the development of melanoma, especially in individuals with pigmented nevi or atypical nevi.

Meanwhile, oncolytic viruses are self-replicating viruses that selectively infect, replicate, and induce apoptosis in cancer cells rather than normal cells, and are being touted as the fourth generation of anticancer agents that will revolutionize the anticancer drug market. The destruction of tumor cells by oncolytic viruses can induce reinfection of surrounding tumor cells, leading to an amplified antitumor effect. Furthermore, oncolytic viruses also have the ability to inhibit neoangiogenesis by infecting vascular endothelial cells in the tumor. However, the development of oncolytic virus immunotherapy is hindered by the high level of difficulty in developing and manufacturing the virus, which creates a significant entry barrier. Thus, the development of oncolytic viruses that can be applied in clinical settings is still in its infancy, and effective oncolytic viruses that target rare cancers have not yet been identified.

### [Disclosure]

### [Technical Problem]

The present invention has been made to solve the above-mentioned problems, and has been completed by confirming that a novel modified reovirus derived from a wild-type reovirus not only has an excellent anticancer effect on various cancers including rare cancers, but also can enhance the effect of anticancer immunotherapy.

Therefore, an object of the present invention is to provide a modified reovirus having an anticancer effect in cancer showing resistance to wild-type reovirus.

Another object of the present invention is to provide a pharmaceutical composition for the prevention or treatment of cancer comprising the above-mentioned modified reovirus as an active ingredient.

Yet another object of the present invention is to provide a pharmaceutical composition for co-administration with an immune checkpoint inhibitor comprising the above-mentioned modified reovirus as an active ingredient.

However, the technical problem that the present invention aims to solve is not limited to the above-mentioned problems, and other problems not mentioned will be clearly understood by those skilled in the art of the technical field to which the present invention belongs from the following description.

### [Technical Solution]

The present invention provides a modified reovirus comprising: a mutation in which amino acids 251 to 455 are deleted from the amino acid sequence of SEQ ID NO: 1; and
one or more mutations selected from the group consisting of a mutation in which the 963^{rd} Met of the amino acid sequence of SEQ ID NO: 2 is substituted with Val, and a mutation in which the 1265^{th} Thr of the amino acid sequence of SEQ ID NO: 2 is substituted with Ile.

In one embodiment of the present invention, the modified reovirus may further comprise a mutation in which the 227^{th} Ile of the amino acid sequence of SEQ ID NO: 1 is substituted with Val, but is not limited thereto.

In another embodiment of the present invention, the modified reovirus may further comprise one or more mutations selected from a group consisting of, but not limited to:
(a) Glu at the 73^{th} position of the amino acid sequence of SEQ ID NO: 3 being substituted with Asp;
(b) Asp at the 434^{th} position of the amino acid sequence of SEQ ID NO: 3 being substituted with Asn; and
(c) Val at the 644^{th} position of the amino acid sequence of SEQ ID NO: 3 being substituted with Ala.

In yet another embodiment of the present invention, the modified reovirus may further comprise one or more mutations selected from a group consisting of, but not limited to:
(a) Lys at the 64^{th} position of the amino acid sequence of SEQ ID NO: 4 being substituted with Glu;
(b) Ser at the 177^{th} position of the amino acid sequence of SEQ ID NO: 4 being substituted with Phe;
(c) Glu at the 229^{th} position of the amino acid sequence of SEQ ID NO: 4 being substituted with Asp; and
(d) His at the 251^{th} position of the amino acid sequence of SEQ ID NO: 4 being substituted with Leu.

In one embodiment of the present invention, the modified reovirus may be derived from a wild-type human reovirus, but is not limited thereto.

The present invention also provides a pharmaceutical composition for cancer prevention or treatment comprising the modified reovirus as an active ingredient.

Furthermore, the present invention provides a cancer prevention or treatment method comprising administering the modified reovirus to a subject in need thereof.

Additionally, the present invention provides the use of the modified reovirus for cancer prevention or treatment.

Moreover, the present invention provides the use of the modified reovirus for the manufacture of an anticancer drug.

Furthermore, the present invention provides a kit for cancer prevention or treatment comprising the composition according to the present invention.

In one embodiment of the present invention, the cancer may be selected from the group consisting of squamous cell carcinoma, neuroblastoma, lung cancer, adenocarcinoma of the lung, peritoneal cancer, skin cancer, ocular cancer, rectal cancer, perianal cancer, esophageal cancer, small intestine cancer, endocrine gland cancer, parathyroid cancer, adrenal cancer, osteosarcoma, soft tissue sarcoma, urethral cancer, blood cancer, liver cancer, gastrointestinal cancer, pancreatic cancer, glioblastoma, cervical cancer, ovarian cancer, bladder cancer, breast cancer, colon cancer, large intestine cancer, endometrial cancer, uterine cancer, salivary gland cancer, kidney cancer, prostate cancer, vulvar cancer, thyroid cancer, head and neck cancer, oral cancer, tongue cancer, brain cancer, and stromal tumors, but is not limited thereto.

In another embodiment of the present invention, the cancer may be a cancer resistant to taxane-based anticancer agents, but is not limited thereto.

In yet another embodiment of the present invention, the taxane-based anticancer agent may be selected from a group consisting of paclitaxel, larotaxel, cabazitaxel, docetaxel, ortataxel, and tesetaxel, but is not limited thereto.

In yet another embodiment of the present invention, the modified reovirus may be included in the composition at a dose of 1×10⁵ to 1×10²⁰ TCID50, but is not limited thereto.

In yet another embodiment of the present invention, the composition may be for direct administration into a tumor or intravenous administration, but is not limited thereto.

In yet another embodiment of the present invention, the composition may be administered more than once to a subject in need thereof, but is not limited thereto.

In yet another embodiment of the present invention, the composition may be co-administered with a wild-type reovirus, but is not limited thereto.

In yet another embodiment of the present invention, the composition may be administered before or after administration of the wild-type reovirus, but is not limited thereto.

In yet another embodiment of the present invention, the composition may further comprise an immune checkpoint inhibitor as an active ingredient, but is not limited thereto.

In yet another embodiment of the present invention, the immune checkpoint inhibitor may be selected from a group consisting of PD-1 inhibitors, PD-L1 inhibitors, PD-L2 inhibitors, OX40 inhibitors, CTLA-4 inhibitors, 4-1BB inhibitors, LAG-3 inhibitors, B7-H4 inhibitors, HVEM inhibitors, TIM4 inhibitors, GAL9 inhibitors, VISTA inhibitors, KIR inhibitors, TIGIT inhibitors, and BTLA inhibitors, but is not limited thereto.

In yet another embodiment of the present invention, the composition may be in the form of a mixture in which the modified reovirus and the immune checkpoint inhibitor are mixed, but is not limited thereto.

In yet another embodiment of the present invention, the composition may be in the form of separate formulations of the modified reovirus and the immune checkpoint inhibitor, to be administered simultaneously or sequentially, but is not limited thereto.

Additionally, the present invention provides a pharmaceutical composition for combination therapy with an immune checkpoint inhibitor, comprising the modified reovirus as an active ingredient.

In one embodiment of the present invention, the composition may be administered simultaneously, separately, or sequentially with the immune checkpoint inhibitor, but is not limited thereto.

Furthermore, the present invention provides a method for preventing or treating cancer, comprising administering a composition including both the modified reovirus and the immune checkpoint inhibitor to a subject in need thereof.

Additionally, the present invention provides a use of the modified reovirus for combination therapy with an immune checkpoint inhibitor.

Moreover, the present invention provides a use of the modified reovirus for the manufacture of a medicament for combination therapy with an immune checkpoint inhibitor.

### [Advantageous Effects]

The present invention relates to a modified reovirus and its use, wherein the novel modified reovirus derived from a wild-type reovirus not only exhibits excellent anticancer effects against various cancers, including rare cancers, but also enhances the efficacy of immunotherapeutic agents. Specifically, the modified reovirus according to the present invention significantly reduced the survival rate of all cancer cell lines, including various types of cancer cells including rare cancer cells such as melanoma cells, tongue cancer cells, etc., when treated with the modified reovirus, and in particular, was found to have excellent anticancer effects against taxane-based anticancer agent-resistant cancer cell lines. Furthermore, the modified reovirus according to the present invention demonstrated dose-dependent anticancer effects in melanoma, bladder cancer, and oral cancer mouse models, regardless of the route of administration, and the anticancer effects were further increased when administered repeatedly or in combination with wild-type reovirus. Notably, it was confirmed that the modified reovirus according to the present invention synergistically enhanced anticancer effects when used in combination with immune checkpoint inhibitors. Therefore, the modified reovirus according to the present invention is expected to be useful as a new anticancer therapy for treating rare cancers and as a combination drug for immunotherapeutic agents.

### [Description of the Drawings]

Figure 1A shows the results of cell survival rates according to treatment concentrations after treating normal cell lines with paclitaxel or modified reovirus (RP116) at various concentrations.
Figure 1B shows the results of cancer cell survival rates according to treatment concentrations after treating various paclitaxel-resistant cancer cell lines with paclitaxel or RP116 at various concentrations.
Figure 2A shows the results of cancer cell survival rates and IC₅₀ values according to treatment concentrations after treating various human-derived cancer cell lines with RP116 at various concentrations.
Figure 2B is a table summarizing cell death rates according to treatment concentrations after treating normal cell lines and various types of cancer cell lines with RP116 at various concentrations.
Figure 3 shows the results of crystal violet staining for cancer cell survival levels according to treatment concentrations after treating various skin cancer cell lines with wild-type reovirus or RP116 (Mock: untreated control group, the same applies hereafter).
Figure 4 shows the results of crystal violet staining for cancer cell survival levels according to treatment concentrations after treating various oral cancer cell lines with wild-type reovirus or RP116.
Figure 5 shows the results of cancer cell survival rates and IC₅₀ values according to treatment concentrations after treating various oral cancer cell lines with wild-type reovirus or RP 116.
Figure 6A is a diagram showing the method of intratumoral (IT) or intravenous (IV) administration of modified reovirus to skin cancer mouse models.
Figure 6B shows the results of the average tumor volume changes for each group over time after administering RP116 at 1×10⁸ TCID50 or 1×10⁹ TCID50 doses intratumorally or intravenously to skin cancer mouse models (Control: untreated control group, the same applies hereafter).
Figure 6C shows the results of the average body weight changes for each group over time after administering RP116 at 1×10⁸ TCID50 or 1×10⁹ TCID50 doses intratumorally or intravenously to skin cancer mouse models.
Figure 7A shows the results of the average tumor volume changes for each group over time after administering RP116 at a 1×10⁹ TCID50 dose 2 or 5 times intravenously to skin cancer mouse models (Vehicle: untreated control group, the same applies hereafter).
Figure 7B shows the results of the survival rate changes for each group over time after administering RP116 at a 1×10⁹ TCID50 dose 2 or 5 times intravenously to skin cancer mouse models.
Figure 7C shows the results of the average body weight changes for each group over time after administering RP116 at a 1×10⁹ TCID50 dose 2 or 5 times intravenously to skin cancer mouse models.
Figure 8A shows the results of the average tumor volume changes for each group over time after administering RP116 alone or in combination with an immunotherapeutic agent (αPD-L1) to skin cancer mouse models.
Figure 8B shows the results of the survival rate changes for each group over time after administering RP116 alone or in combination with an immunotherapeutic agent to skin cancer mouse models.
Figure 9A shows the results of the average tumor volume changes for each group over time after administering RP116 to oral cancer mouse models.
Figure 9B shows the results of the average body weight changes for each group over time after administering RP116 to oral cancer mouse models.
Figure 10A shows the results of the change in average tumor volume over time for each group, wherein wild-type reovirus was administered continuously in a skin cancer mouse model (WT/WT), or RP116 was administered after administering the wild-type reovirus (WT/RP116).
Figure 10B shows the results of the change in average tumor volume over time for each group after administering RP116 consecutively (RP116/RP116) or administering wild-type reovirus after RP116 treatment (RP116/WT) in skin cancer mouse models.
Figure 11A is an experimental schematic for determining the resistance to neutralizing antibodies against RP116 or wild-type reovirus (RC402).
Figure 11B shows the results of cell survival rates (y-axis) according to the dilution multiples of neutralizing antibodies after treating cells with RC402 or RP116 virus and RC402-induced neutralizing antibody (RC402 ab).
Figure 11C shows the results of cell survival rates (y-axis) according to the dilution multiples of neutralizing antibodies after treating cells with RC402 or RP116 virus and RP116-induced neutralizing antibody (RP116 ab).

### [Best Modes of the Invention]

The present invention relates to a modified reovirus and its use, wherein a novel modified reovirus derived from wild-type reovirus not only exhibits excellent anticancer effects against various cancers, including rare cancers, but also has been confirmed to enhance the effects of immunotherapeutic agents.

Specifically, in one embodiment of the present invention, a modified reovirus (RP116) was prepared and its molecular biological properties were confirmed (Examples 1 and 2).

In another embodiment of the present invention, after treating normal cells and cancer cell lines having resistance to paclitaxel, which is a taxane-based anticancer drug, with RP116, it was confirmed that the modified reovirus according to the present invention exerts a specific anticancer effect on taxane anticancer drug-resistant cancer cells without toxicity to normal cells (Example 3).

In yet another embodiment of the present invention, after treating various cancer cell lines, including rare cancers, with RP116, it was confirmed that the modified reovirus according to the present invention exhibits dose-dependent anticancer effects against multiple types of cancer (Example 4).

In yet another embodiment of the present invention, after treating various skin cancer cell lines, oral cancer cell lines, and bladder cancer cell lines with wild-type reovirus and RP116, respectively, it was confirmed that the modified reovirus according to the present invention has more potent anticancer effects than wild-type reovirus (Examples 5 to 7).

In yet another embodiment of the present invention, after administering RP116 at low or high doses directly into tumors or intravenously in skin cancer mouse models, it was confirmed that the modified reovirus according to the present invention showed excellent anticancer effects in both administration routes, and that the anticancer effects were correlated with the virus dose (Example 8).

In yet another embodiment of the present invention, after comparing the anticancer effects of RP116 in skin cancer mouse models depending on the number of administrations, it was confirmed that the more times the modified reovirus according to the present invention was administered, the more the anticancer effects were enhanced (Example 9).

In yet another embodiment of the present invention, after administering the immune checkpoint inhibitor in combination with RP116 in skin cancer mouse models, it was confirmed that the modified reovirus according to the present invention not only exerts excellent anticancer effects on its own, but also synergistically enhances the anticancer effects when combined with immunotherapeutic agents (Example 10).

In yet another embodiment of the present invention, after administering RP116 in an oral cancer mouse model, it was confirmed that the modified reovirus according to the present invention has also a potent tumor growth inhibitory effect in oral cancer (Example 11).

In yet another embodiment of the present invention, after examining the enhancement of the anticancer effects of the virus when RP116 and wild-type reovirus were administered in a cross-over manner (RP116→WT, or WT→RP116) in skin cancer mouse models, it was confirmed that the anticancer effects were further enhanced when the modified reovirus according to the present invention and wild-type reovirus were administered in a cross-over manner compared to consecutive administration of the same type of virus (Example 12).

In yet another embodiment of the present invention, after confirming the resistance of RP116 to neutralizing antibodies induced by RP116 or wild-type reovirus, it was confirmed that the modified reovirus according to the present invention has strong resistance to both types of neutralizing antibodies (Example 13).

Through the above embodiments, it has been confirmed that the modified reovirus according to the present invention exhibits a specific anticancer effect against various cancers, including rare cancers such as melanoma and tongue cancer, and that this anticancer effect is superior to that of wild-type reovirus. Furthermore, the modified reovirus not only effectively suppresses tumor growth in animal models upon both intratumoral and intravenous administration, allowing for a flexible choice of administration routes depending on the type of cancer, but also further enhances the anticancer effect through repeated administration or cross-administration with wild-type reovirus. In addition, the modified reovirus exhibits a more potent anticancer effect when combined with immune anticancer agents and demonstrates high resistance to neutralizing antibodies, a weakness of anticancer viruses. Therefore, the modified reovirus according to the present invention is expected to be utilized as a new anticancer therapy for the treatment of rare cancers and as a combination drug with immune anticancer agents.

Hereinafter, the present invention will be described in detail.

The present invention provides a modified reovirus that exhibits anticancer effects in cancers resistant to wild-type reovirus. The modified reovirus is characterized by not only having a defect in the antigenic determinant of the sigma-1 protein but also including additional sequence variants in structural proteins such as lambda-2, which constitute the outer capsid of the virus particle.

The reovirus (respiratory enteric orphan virus, REO virus) is a non-enveloped icosahedral virus possessing double-stranded RNA segments as its genome. The reovirus is commonly isolated from healthy human gastrointestinal and respiratory systems and is considered to be a non-pathogenic virome. The reovirus is known as an oncolytic virus capable of infecting and killing various transformed cells. Therefore, the reovirus, like other typical oncolytic viruses, has the advantage of having low side effects, as it can specifically infect and induce the death of cancer cells without significantly affecting normal cells. Additionally, after proliferating in primarily infected cancer cells, the virus can infect surrounding and distant cancer cells, resulting in a broad anti-cancer effect.

However, wild-type reovirus may have their anti-cancer functions weakened by neutralizing antibodies when injected into the body, and the anti-cancer functions of the virus may be suppressed by factors such as the tumor microenvironment of cancer cells. Furthermore, there still exists the issue of the reovirus infecting normal cells and causing abnormalities in the host. To address the host toxicity problem of wild-type reovirus, the inventors have developed an attenuated reovirus (AV) that expresses a truncated form of the Sigma 1 protein due to the presence of a premature stop codon in the wild-type Sigma 1 protein-coding gene. The attenuated reovirus has been found to have reduced host toxicity compared to wild-type reovirus, but its anti-cancer effect only maintained the level of tumor lysis equivalent to that of wild-type reovirus. In response, the inventors conducted research on a modified reovirus with even lower toxicity to normal cells compared to wild-type reovirus, while exerting a stronger anti-cancer effect. The modified reovirus according to the present invention is characterized by having no toxicity to normal cells compared to wild-type reovirus while being able to more powerfully infect and kill cancer cells.

Therefore, the modified reovirus according to the present invention is characterized by the polypeptide coded by the S1 segment (SEQ ID NO: 1; Sigma-1 protein) lacking amino acids 251 to 455, the polypeptide coded by the L2 segment (SEQ ID NO: 2; Lambda-2 protein) having the 963^{rd} Met replaced with Val and/or the 1265^{th} Thr replaced with Ile. In other words, the modified reovirus according to the present invention is characterized by including a substitution mutation in the Lambda-2 protein compared to wild-type reovirus and attenuated reovirus. In the present invention, the Sigma-1 protein is a protein involved in the attachment of the virus to cells and corresponds to the major antigen determinant of the virus particles. In the present invention, the Lambda-2 protein is an outer capsid protein involved in the mRNA capping of reovirus.

Moreover, the modified reovirus according to the present invention may further comprise a mutation wherein the 227^{th} Ile (Isoleucine) in the amino acid sequence of SEQ ID NO: 1 is replaced with Val (Valine).

Additionally, the modified reovirus according to the present invention may further comprise additional mutations in the polypeptide coded by the M2 segment (SEQ ID NO: 3; Mu-1 protein). Specifically, the modified reovirus may further comprise one or more mutations selected from the group consisting of:
(a) the 73^{rd} Glu in the amino acid sequence of SEQ ID NO: 3 being replaced with Asp;
(b) the 434^{th} Asp in the amino acid sequence of SEQ ID NO: 3 being replaced with Asn; and
(c) the 644^{th} Val in the amino acid sequence of SEQ ID NO: 3 being replaced with Ala.

In the present invention, the Mu-1 protein is a major outer capsid protein involved in the penetration of the virus into host cell membranes.

Furthermore, the modified reovirus according to the present invention may further comprise additional mutations in the polypeptide coded by the S4 segment (SEQ ID NO: 4; Sigma-3 protein). Specifically, the modified reovirus may further comprise one or more mutations selected from the group consisting of
(a) the 64^{th} Lys in the amino acid sequence of SEQ ID NO: 4 being replaced with Glu;
(b) the 177^{th} Ser in the amino acid sequence of SEQ ID NO: 4 being replaced with Phe;
(c) the 229^{th} Glu in the amino acid sequence of SEQ ID NO: 4 being replaced with Asp; and
(d) the 251^{st} His in the amino acid sequence of SEQ ID NO: 4 being replaced with Leu.

Additionally, the modified reovirus according to the present invention may further comprise a mutation wherein the 399^{th} Ile in the amino acid sequence of the polypeptide coded by the L1 segment (SEQ ID NO: 5; Lambda-3 protein) is replaced with Thr and/or the 1202^{nd} Lys in the amino acid sequence of the polypeptide coded by the L1 segment (SEQ ID NO: 5; Lambda-3 protein) is replaced with Glu.

Moreover, the modified reovirus according to the present invention may further comprise a mutation wherein the 16^{th} Gly in the amino acid sequence of the polypeptide coded by the L3 segment (SEQ ID NO: 6; Lambda-1 protein) is replaced with Val.

Additionally, the modified reovirus according to the present invention may further comprise a mutation wherein the 478^{th} Ile in the amino acid sequence of the polypeptide coded by the M3 segment (SEQ ID NO: 7; MuNS protein) is replaced with Leu and/or the 657^{th} Thr in the amino acid sequence of the polypeptide coded by the M3 segment (SEQ ID NO: 7; MuNS protein) is replaced with Ala.

Furthermore, the modified reovirus according to the present invention may further include a mutation in which the 50^{th} Arg in the amino acid sequence of the polypeptide encoded by the S2 segment (SEQ ID NO: 8; Sigma-2 protein) is replaced with Lys.

The nucleotide sequences of each gene segment of the modified reovirus according to the present invention and the amino acid sequences of the proteins encoded by them are listed in the sequence listing of the present specification.

In the present invention, a gene (nucleic acid molecule) with a specific SEQ ID NO. may include or consist of the nucleotide sequence of the specific SEQ ID NO., and variants of the nucleotide sequence are included within the scope of the present invention as long as the purpose and function of the modified reovirus according to the present invention are maintained. For example, a nucleic acid molecule of a nucleotide sequence indicated by a specific SEQ ID NO. is a concept that includes functional equivalents of the constituent nucleic acid molecules, such as variants that have a partial nucleotide sequence modified by deletion, substitution, or insertion but can functionally act identically to the nucleic acid molecule. Specifically, a nucleic acid molecule indicated by a specific SEQ ID NO. may include nucleotide sequences having 70% or more, more preferably 80% or more, even more preferably 90% or more, and most preferably 95% or more sequence homology with the nucleotide sequence indicated by the specific SEQ ID NO. For example, it includes polynucleotides having sequence homologies of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and 100%. The "% of sequence homology" for polynucleotides is determined by comparing two optimally aligned sequences and a comparison region, and a portion of the polynucleotide sequence in the comparison region may include additions or deletions (i.e., gaps) relative to the reference sequence for the optimal alignment of the two sequences (excluding additions or deletions).

Similarly, a polypeptide (protein) with a specific SEQ ID NO. may include or consist of the amino acid sequence of the specific SEQ ID NO., and variants of the amino acid sequence are included within the scope of the present invention as long as the purpose and function of the modified reovirus according to the present invention are maintained. For example, a polypeptide of an amino acid sequence indicated by a specific SEQ ID NO. is a concept that includes functional equivalents of the constituent polypeptide molecules, such as variants that have a partial amino acid sequence modified by deletion, substitution, or insertion but can functionally act identically to the polypeptide. Specifically, a polypeptide indicated by a specific SEQ ID NO. may include amino acid sequences having 70% or more, more preferably 80% or more, even more preferably 90% or more, and most preferably 95% or more sequence homology with the amino acid sequence indicated by the specific SEQ ID NO. For example, the polypeptides can have a sequence identity of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. The "% of sequence identity" for polypeptides is determined by comparing two optimally aligned sequences within a comparison region, wherein a portion of the amino acid sequence in the comparison region may include additions or deletions (i.e., gaps) relative to the reference sequence (without additions or deletions) for the optimal alignment of the two sequences.

The modified reovirus according to the present invention includes wild-type nucleotide and amino acid sequences identical to those of wild-type reovirus, except for the aforementioned mutations. However, it is obvious that some mutations, such as deletion, substitution, and/or insertion of some nucleotides or amino acids, can occur within the scope of maintaining the function and characteristics of the wild-type sequence due to the nature of the virus. Therefore, the modified reovirus according to the present invention may additionally include variants of the wild-type sequence that do not impair the function (anticancer effect) of the virus, in addition to the aforementioned mutations. The genomic sequences of the wild-type reovirus according to the present invention are described in detail in the sequence listing of this specification.

According to the present invention, a modified reovirus may be derived from any wild-type reovirus and may be a member of the Reovirus family, which can be acquired from various sources. Preferably, the modified reovirus according to the present invention may be derived from a wild-type human reovirus. Preferably, the wild-type reovirus may be selected from human reovirus type 1, human reovirus type 2, and human reovirus type 3. More preferably, the wild-type reovirus may be selected from human reovirus type 1 strain Lang, human reovirus type 2 strain Jones, and human reovirus type 3 strains Dearing or Abney. Most preferably, the wild-type reovirus according to the present invention may be a type 3 reovirus. In addition, the modified reovirus according to the present invention may be derived from one or more reoviruses exhibiting tropism for cells of other mammalian species, including primates (chimpanzees, gorillas, short-tailed monkeys, monkeys, etc.), rodents (mice, rats, gerbils, hamsters, rabbits, guinea pigs, etc.), dogs, cats, and common livestock (cows, horses, pigs, goats) but is not limited thereto.

Furthermore, the present invention provides a pharmaceutical composition for cancer prevention or treatment comprising the modified reovirus according to the present invention as an active ingredient.

As used in this specification, the term "cancer" refers to uncontrolled cellular growth characterized by the formation of a cellular mass called a tumor, which invades surrounding tissues and, in severe cases, metastasizes to other organs of the body. In the present invention, cancer may be a solid cancer or a hematologic cancer and may be selected from the group consisting of squamous cell carcinoma, neuroblastoma, lung cancer, adenocarcinoma of the lung, peritoneal cancer, skin cancer, ocular cancer, rectal cancer, perianal cancer, esophageal cancer, small intestine cancer, endocrine gland cancer, parathyroid cancer, adrenal cancer, osteosarcoma, soft tissue sarcoma, urethral cancer, blood cancer, liver cancer, gastrointestinal cancer, pancreatic cancer, glioblastoma, cervical cancer, ovarian cancer, bladder cancer, breast cancer, colon cancer, large intestine cancer, endometrial cancer, uterine cancer, salivary gland cancer, kidney cancer, prostate cancer, vulvar cancer, thyroid cancer, head and neck cancer, oral cancer, tongue cancer, brain cancer, and stromal tumors. The blood cancer may be leukemia, lymphoma, or multiple myeloma, among others. Preferably, the skin cancer may be selected from squamous cell carcinoma, basal cell carcinoma, and melanoma. Preferably, the melanoma may be metastatic melanoma. In one embodiment of the present invention, the cancer may be a cancer expressing or not expressing PD-L1. In another embodiment of the present invention, the cancer may be a cancer with a mutation in a cancer suppressor gene (p53, Rb, etc.) or an activating mutation in RAS. More preferably, the cancer according to the present invention may be resistant to wild-type reovirus.

Alternatively, in the present invention, the cancer may be a cancer resistant to taxane-based anticancer drugs.

In the present invention, "resistance to anticancer drugs" refers to the absence of therapeutic effect from the beginning of treatment or the loss of therapeutic effect during the continuous treatment process, despite the initial therapeutic effect when using anticancer drugs for cancer treatment. The general criteria for therapeutic effect in anticancer drugs are classified into four categories according to the standards established by the WHO: (1) complete disappearance of the tumor with therapeutic effect lasting for more than 4 weeks (complete response); (2) tumor size reduction of 50% or more (partial response); (3) tumor size reduction of less than 50% (stable disease); and (4) tumor size increase of 25% or more (progressive disease). Thus, according to the WHO criteria, resistance to anticancer drugs means that there is no therapeutic effect from the beginning of treatment, or there is an initial cancer treatment effect ((1) and (2) above) but the cancer treatment effect is lost during the continuous treatment process ((3) and (4) above).

In the present invention, the anticancer drug may be a taxane-based anticancer drug. More preferably, the taxane-based anticancer drug may be selected from the group consisting of paclitaxel, larotaxel, cabazitaxel, docetaxel, ortataxel, and tesetaxel.

The content of the modified reovirus in the composition of the present invention can be adjusted appropriately depending on the symptoms of the disease, the degree of symptom progression, and the patient's condition, for example, from 0.0001 to 99.9 weight% or from 0.001 to 50 weight% based on the total weight of the composition, but is not limited thereto. The above amount ratio is based on the dry weight excluding the solvent.

Preferably, the modified reovirus according to the present invention may be contained in the composition at a dosage of 1×10⁵ to 1×10²⁰ TCID50 (Tissue Culture Infective Dose 50%). For example, the modified reovirus may be contained in the composition at a dosage of 1×10⁵ to 1×10²⁰, 1×10⁵ to 1×10¹⁹, 1×10⁵ to 1×10¹⁸, 1×10⁵ to 1×10¹⁷, 1×10⁵ to 1×10¹⁶, 1×10⁵ to 1×10¹⁵, 1×10⁵ to 1×10¹⁴, 1×10⁵ to 1×10¹³, 1×10⁵ to 1×10¹², 1×10⁵ to 1×10¹¹, 1×10⁵ to 1×10¹⁰, 1×10⁵ to 1×10⁹, 1×10⁵ to 1×10⁸, 1×10⁵ to 1×10⁷, 1×10⁵ to 1×10⁶, 1×10⁶ to 1×10¹⁵, 1×10⁶ to 1×10¹², 1×10⁶ to 1×10¹⁰, 1×10⁷ to 1×10¹⁵, 1×10⁷ to 1×10¹², or 1×10⁷ to 1×10¹⁰ TCID50, but is not limited thereto.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used. The pharmaceutical composition according to the present invention may be formulated such that the modified reovirus contained in the composition is bioavailable when administered to a subject.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields. Preferably, the effective dose of the pharmaceutical composition according to the present invention may be a dosage at which the modified reovirus is administered in an amount of 1×10⁵ to 1×10²⁰ TCID50.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like. Preferably, the modified reovirus according to the present invention may be administered via direct intratumoral injection or intravenous administration, with an appropriate administration method selected based on the condition and type of the tumor.

The pharmaceutical composition according to the present invention may be administered as an individual therapeutic agent or in combination with other therapeutic agents, sequentially or simultaneously with conventional therapeutic agents, and may be administered as a single or multiple doses. It is important to administer an amount that can achieve maximum effect with a minimum amount without side effects, considering all the factors mentioned above, and this can be easily determined by a person of ordinary skill in the art to which the present invention pertains. Preferably, the pharmaceutical composition according to the present invention may be administered more than once to a subject in need thereof (e.g., cancer patients). The inventors have confirmed through specific examples that the anti-cancer effect of the modified reovirus according to the present invention is further enhanced with multiple administrations.

Thus, the composition according to the present invention may not only be administered as a single dose to a subject in need thereof but may also be administered more than twice and repeated until the tumor is reduced or eliminated. For example, the pharmaceutical composition according to the present invention may be administered 2 to 50 times, 2 to 45 times, 2 to 40 times, 2 to 35 times, 2 to 30 times, 2 to 25 times, 2 to 20 times, 2 to 15 times, 2 to 14 times, 2 to 13 times, 2 to 12 times, 2 to 11 times, 2 to 10 times, 2 to 9 times, 2 to 8 times, 2 to 7 times, 2 to 6 times, 2 to 5 times, or 2 to 4 times, but this is not limited to these examples.

Furthermore, the repeated administration may be carried out at intervals of 1 to 100 days. For example, the repeated administration may be carried out at intervals of 1 to 100 days, 1 to 90 days, 1 to 80 days, 1 to 70 days, 1 to 60 days, 1 to 50 days, 1 to 40 days, 1 to 30 days, 1 to 20 days, 1 to 15 days, 1 to 10 days, 1 to 9 days, 1 to 8 days, 1 to 7 days, 1 to 6 days, or 1 to 5 days, but this is not limited to these examples.

Additionally, the composition according to the present invention may be co-administered with a wild-type reovirus. In the context of the present invention, "crossover administration" refers to the administration of two or more drugs alternately at a fixed or unspecified interval. The inventors have confirmed through specific examples that the anti-cancer effect is increased when the modified reovirus according to the present invention is co-administered with a wild-type reovirus compared to administering the same reovirus continuously. There is no restriction on the order of co-administration of the composition according to the present invention (i.e., the modified reovirus according to the present invention) and the wild-type reovirus. That is, the composition according to the present invention may be administered first followed by the wild-type reovirus, or vice versa.

Alternatively, the pharmaceutical composition according to the present invention may further comprise a wild-type reovirus in addition to the modified reovirus according to the present invention. Preferably, the composition may be in the form of the modified reovirus and the wild-type reovirus being separately formulated and sequentially administered.

In the present invention, "subject" refers to a target in need of treatment for a disease, more specifically, mammals such as humans or non-human primates, mice, rats, dogs, cats, horses, and cows.

In the present invention, "administration" refers to providing a predetermined composition of the present invention to a subject by any appropriate means.

In the present invention, "prevention" refers to all actions that suppress or delay the onset of a target disease, "treatment" refers to all actions in which the target disease and its associated metabolic abnormalities are improved or favorably altered by the administration of a pharmaceutical composition according to the present invention, and "improvement" refers to all actions in which parameters related to the target disease, for example, the severity of symptoms, are reduced by the administration of a composition according to the present invention.

According to the present invention, the pharmaceutical composition may further comprise an immunotherapeutic anticancer agent in addition to the modified reovirus. The immunotherapeutic anticancer agent is an anticancer agent that exerts anticancer effects by activating the immune system and enhancing specificity, memory, and adaptiveness. The inventors have confirmed, through specific embodiments, that the anticancer effects of the modified reovirus and immunotherapeutic anticancer agent according to the present invention are further enhanced when used in combination. In the present invention, the immunotherapeutic anticancer agent may be selected from immune checkpoint inhibitors, immune cell therapies, anticancer vaccines, and antibody-drug conjugates, but is not limited thereto.

Preferably, the immunotherapeutic anticancer agent may be an immune checkpoint inhibitor. An immune checkpoint is an immune response-regulating protein expressed on the surface of normal cells, which weakens excessive immune responses and suppresses harmful and indiscriminate autoimmune reactions to protect normal tissues. However, some cancer cells express immune checkpoint proteins, interact with immune cells, and inactivate their immune functions, thereby neutralizing anticancer immune responses. Immune checkpoint inhibitors target immune checkpoint proteins expressed on cancer cells and interfere with their interaction with immune cells, thereby blocking cancer cell immune evasion.

There is no restriction on the specific types of immune checkpoint inhibitors, but preferably, they may be selected from a group consisting of PD-1 inhibitors, PD-L1 inhibitors, PD-L2 inhibitors, OX40 inhibitors, CTLA-4 inhibitors, 4-1BB inhibitors, LAG-3 inhibitors, B7-H4 inhibitors, HVEM inhibitors, TIM4 inhibitors, GAL9 inhibitors, VISTA inhibitors, KIR inhibitors, TIGIT inhibitors, and BTLA inhibitors. Preferably, the immune checkpoint inhibitors according to the present invention may be small molecules, ligands, or macromolecules targeting the immune checkpoint proteins, and more preferably, antibodies targeting the immune checkpoint proteins. Alternatively, the immune checkpoint inhibitors according to the present invention may be selected from ipilimumab, pembrolizumab, nivolumab, cemiplimab, atezolizumab, avelumab, and durvalumab.

According to the present invention, the composition may be in the form of a mixture in which the modified reovirus and the immune checkpoint inhibitor are mixed.

Alternatively, the composition according to the present invention may be in a form in which the modified reovirus and the immune checkpoint inhibitor are each formulated for simultaneous or sequential administration. In this case, the composition may be a pharmaceutical composition for combination administration for simultaneous or sequential administration, including a first pharmaceutical composition containing a pharmaceutically effective dose of the modified reovirus; and a second pharmaceutical composition containing a pharmaceutically effective dose of the immune checkpoint inhibitor. At this time, for sequential administration, there is no restriction on the order of administration, and the administration method may be appropriately adjusted according to the patient's condition. That is, if the pharmaceutical composition is for sequential combination therapy, the composition may involve administering the modified reovirus ("the first component") first, followed by the immune checkpoint inhibitor ("the second component"), and the reverse order is also possible. The modified reovirus and the immune checkpoint inhibitor according to the present invention may be administered through separate routes. For example, the modified reovirus according to the present invention may be administered intratumorally or intravenously, and the immune checkpoint inhibitor may be administered intraperitoneally.

Furthermore, the present invention provides a kit for cancer prevention or treatment comprising the modified reovirus according to the present invention as an active ingredient. The kit may further comprise a wild-type reovirus and/or an immunotherapeutic anticancer agent. The kit refers to a combination of substances or devices that can be used for cancer prevention or treatment, and it may be in any form suitable for manufacturing, storing, or administering the modified reovirus, without restriction to a specific form. Therefore, the kit according to the present invention can include, without limitation, not only the modified reovirus, wild-type reovirus, and immunotherapeutic anticancer agents but also any components of a kit for treating a specific disease that are known in the art.

Furthermore, the present invention provides a pharmaceutical composition for combination therapy with an immune checkpoint inhibitor, comprising the modified reovirus according to the present invention as an active ingredient. The immunotherapeutic anticancer agent is preferably an immune checkpoint inhibitor.

In this specification, the term "combination therapy" refers to a method of administering individual components of a therapeutic regimen simultaneously, sequentially, or individually. Combination therapy may involve administering two or more drugs simultaneously or sequentially, or alternating administration at fixed or unspecified intervals, among other methods. Although not limited to this, combination therapy can be defined as providing a therapeutically superior and synergistic effect when the efficacy, as measured by response magnitude, response rate, disease progression time, or survival time, is greater than the efficacy obtainable by administering one or the remaining components of the combination therapy at a conventional dosage.

The pharmaceutical composition for combination therapy according to the present invention can enhance the anticancer effects of the immunotherapeutic anticancer agent and sustain the effects. Here, "enhancing the anticancer effect" refers to all effects that can ultimately strengthen the function of the immunotherapeutic anticancer agent, including enhancing the anticancer effect of the anticancer agent, such as tumor growth inhibition, tumor metastasis inhibition, tumor recurrence inhibition, as well as further enhancing the anticancer immune response or suppressing the resistance or tolerance formation of cancer cells to the immunotherapeutic anticancer agent, ultimately enhancing the anticancer effect. Also, "sustaining the effect" includes the concept that the anticancer immune effect on cancer cells is maintained even after complete tumor regression.

In the present invention, the pharmaceutical composition for combination administration can be administered simultaneously with, separately from, or sequentially to the immunotherapeutic anticancer agent, and there is no restriction on the order of administration even when administered sequentially with the immunotherapeutic anticancer agent. However, the administration method may be appropriately adjusted according to the type of cancer, type of anticancer agent, and the patient's condition.

Throughout the specification of the present invention, when a part is said to "comprise" a certain component, it means that it can further include other components unless there is a specific indication to the contrary, rather than excluding other components. The terms of degree used throughout the specification of the present invention, such as "about," "substantially," etc., are used with a meaning close to or at the numerical value when inherent manufacturing and material tolerances are presented for the mentioned meaning and are used to prevent unfair exploitation of the disclosure by unscrupulous infringers who might rely on precise or absolute numerical values to aid understanding of the invention.

Throughout the specification of the present invention, the term "combination of these" included in the Markush-type expression refers to one or more mixtures or combinations selected from the group consisting of the components listed in the Markush-type expression, meaning that it includes one or more selected from the group consisting of said components.

Hereinafter, preferred embodiments are provided to facilitate understanding of the present invention. However, the following embodiments are provided for a better understanding of the invention and are not intended to limit the scope of the invention.

### [Examples]

### Example 1. Preparation of the modified reovirus

In accordance with the present invention, a modified reovirus RP116 is produced by inducing genetic segment reassortment and adaptation between the virus and host cells through co-infection of cell-line having resistance to wild-type reovirus with attenuated reovirus (AV; Br J Cancer. 2011 Jan 18;104(2):290-9) and wild-type reovirus, which has been obtained from the Korea Bank for Pathogenic Viruses (KBPV-VR-38).

Specifically, U-2OS cells (KCLB, 300096), resistant to wild-type reovirus, were treated in a 12-well plate at 3×10⁵ cells/well, and cultured for 24 hours at 37°C and 5% CO₂ incubator to allow the cells to adhere well to the bottom. The wild-type reovirus and the attenuated reovirus AV were mixed at a 1:1 ratio and diluted using DMEM complete media to 10-5, 10-6, and 10-7. Each dilution was treated with 100 µl in each well of two 12-well plates. After 18 hours of virus treatment, the DMEM complete media in the well plate was removed, and 1 ml of 1% Agar was added to each well. Plaques were observed daily under a microscope, marked, and visually identifiable plaques that grew rapidly were collected using a 1 ml pipette. The collected plaques were mixed and rapidly growing plaques were separated again in U-2 OS cells and expanded in BHK21 cells. To characterize the selected and expanded plaques, RNA was extracted from the virus using the PureLink^{™} Viral RNA/DNA Mini kit (Invitrogen, USA) according to the manufacturer's instructions, and the S1 segment, a primary difference between the wild-type virus and AV, was selectively amplified using primer pairs 5'-ATGGATCCTCGCCTACGTKAAGAAG-3', 5'-CTGATCCTCACGTGAAACTACGC-3', and the AccuPower RT-PCR PreMix (Bioneer, Cat #. K-2057) kit, and nucleotide sequencing was performed. Out of the 137 plaque samples analyzed, 37 were found to contain a single S1 segment sequence, all of which had an S1 gene with a stop codon. The 37 mutant strains primarily selected were treated with serial dilutions in 96-well plates with human colorectal cancer cell lines Lovo and DLD1, and normal skin cell HS27, and cultured for 3 days. The survival rate of the treated cells was analyzed by treating with WST reagent, and the most effective and safe mutant strain was selected as the modified reovirus according to the invention. The selected modified reovirus showed the highest cell growth inhibition ability in colorectal cancer cell lines LoVo and DLD1, while human normal cells HS27 showed almost no cell death even when treated with the virus at up to 60,000 MOI based on the number of virus particles. A complete nucleotide sequence analysis was performed for the selected mutant strain.

### Example 2. Verification of molecular biological characteristics of the modified reovirus

RNA was extracted from the selected mutant strain using the PureLinkTM Viral RNA/DNA Mini kit (Invitrogen, USA) and the entire nucleotide sequence data of the virus was obtained through Next Generation Sequencing (NGS). The mutation detection process in the entire nucleotide sequence was carried out using the GATK best practice standardization guidelines, and the novelty of the mutations causing changes in the polypeptide sequences was confirmed by comparing with the data of Reovirus type 3 (strain Dearing) (T3D) reported in the Genebank Database.

As a result, the characteristic amino acid mutations of the modified reovirus according to the present invention were found to be distributed in the loss of amino acid sequences 251 to 455 of the Sigma-1 protein (SEQ ID NO: 1), which corresponds to the major antigenic determinant of the virus particles, the carboxy-terminal of the Lambda-2 protein (SEQ ID NO: 2) interacting with the Sigma-1 protein, and the structural proteins of the outer capsid, Mu-1 (SEQ ID NO: 3) and Sigma-3 protein (SEQ ID NO: 4).

**[Table 1]**

| Segment | Protein Name | Amino Acid Mutation |
|---|---|---|
| L1 | Lambda-3 | Ile399Thr, Lys1202Glu |
| L2 | Lambda-2 | Met963Val, Thr1265Ile |
| L3 | Lambda-1 | Gly 16Val |
| M2 | Mu1 | Glu73Asp, Asp434Asn, Val644Ala |
| M3 | MuNS | Ile478Leu, Thr657Ala |
| S1 | Sigma-1 | Ile227Val, Gln251 STOP |
| S2 | Sigma-2 | Arg50Lys |
| S4 | Sigma-3 | Lys64Glu, Ser177Phe, Glu229Asp, His251Leu |

### Example 3. Inhibition of paclitaxel-resistant cancer cell growth by the modified reovirus

Paclitaxel is a chemotherapeutic agent widely used for various types of cancer, including lung, ovarian, and breast cancer. However, some cancer cells are known to exhibit resistance to paclitaxel, resulting in a reduced cell death effect. Therefore, it was confirmed whether the modified reovirus RP116 according to the present invention has an anticancer effect on paclitaxel-resistant cancer cells.

The cell lines used in this example are as follows: normal cell line HS27, and paclitaxel-resistant cancer cell lines Skmel28, A375, and B16F10. Each cell line was cultured in DMEM medium containing 10% fetal bovine serum and seeded at a density of 3,000 cells/well in a 96-well plate, followed by incubation at 37°C and 5% CO₂ for 24 hours. RP116 was serially diluted at concentrations ranging from 0.008 to 1,000 MOI (Multiplicity of Infection) and treated to the prepared cells, and further incubated for three days at 37°C and 5% CO₂. The number of surviving cells was measured using a WST kit (DoGen, Seoul, South Korea, Cat No: EZ-3000) according to the manufacturer's instructions.

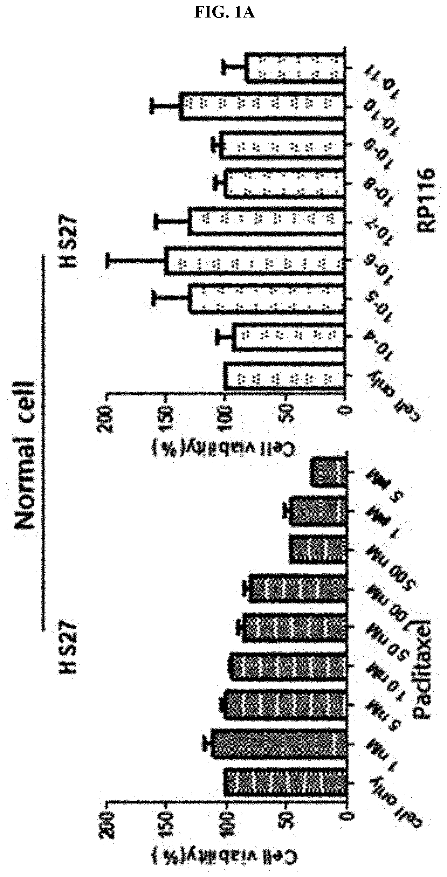

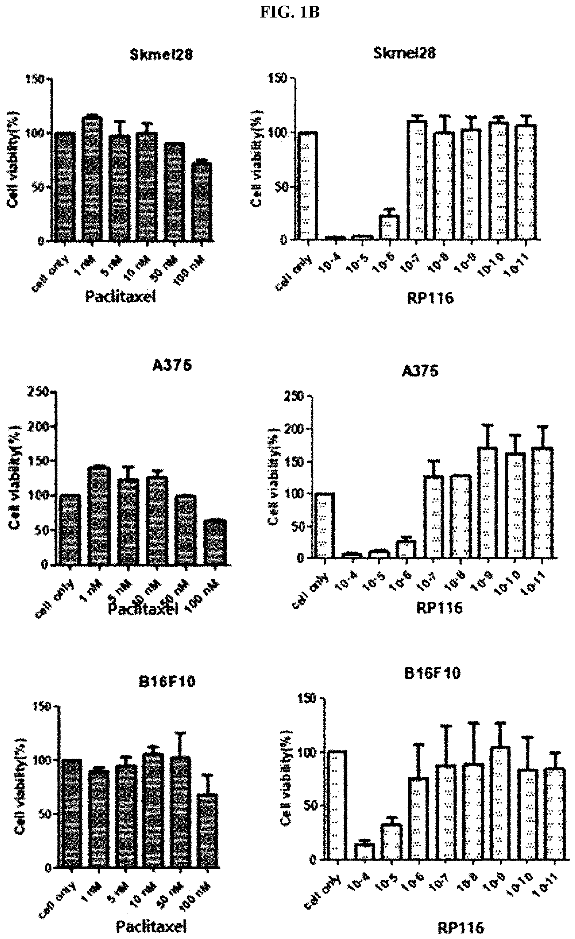

As a result, when paclitaxel was treated to normal cell lines, cell viability decreased in a concentration-dependent manner compared to the control group, confirming the toxicity of paclitaxel in cells. In contrast, no significant difference in cell viability was observed between the control group and the RP116-treated group, indicating that RP116 has no toxicity to normal cells (Figure 1A). On the other hand, an outstanding anticancer effect of RP116 was observed in cancer cells (Figure 1B). Although paclitaxel showed no significant difference in cell viability compared to the control group even at high concentrations of 100 nM or more, RP116 exhibited a cancer cell growth inhibitory effect in all cancer cell lines, and a concentration-dependent cell death effect was observed, confirming a dose-response relationship. The experimental results demonstrate that the modified reovirus according to the present invention has no toxicity to normal cells while having a specific anticancer effect on paclitaxel-resistant cancer cells.

### Example 4. Confirmation of growth inhibitory effects of modified reovirus on various cancer cell lines

Next, it was determined whether the modified reovirus according to the present invention exerts an anticancer effect on various types of human-derived cancer cell lines.

Human-derived colon cancer cell lines (LoVo, HCT116, and DLD-1), skin cancer cell lines (A431), glioma cell lines (SNU489, U87-MG, and SNU466), breast cancer cell lines (MCF7), cervical cancer cell lines (SiHa, HeLa, and ME-180), and liver cancer cell lines (Hep3B) were used for the experiment. Each cell line was cultured in DMEM medium containing 10% fetal bovine serum, seeded at a concentration of 3,000 cells/well in a 96-well plate, and incubated for 24 hours at 37 °C and 5% CO₂. RP116, continuously diluted to concentrations ranging from 0.008 to 1,000 MOI, was treated to the prepared cells, and further incubated for 3 days at 37 °C and 5% CO₂. The number of surviving cells was measured using a WST analysis kit according to the manufacturer's instructions. IC₅₀ values were calculated using nonlinear regression in Prism GraphPad 9.1.0.

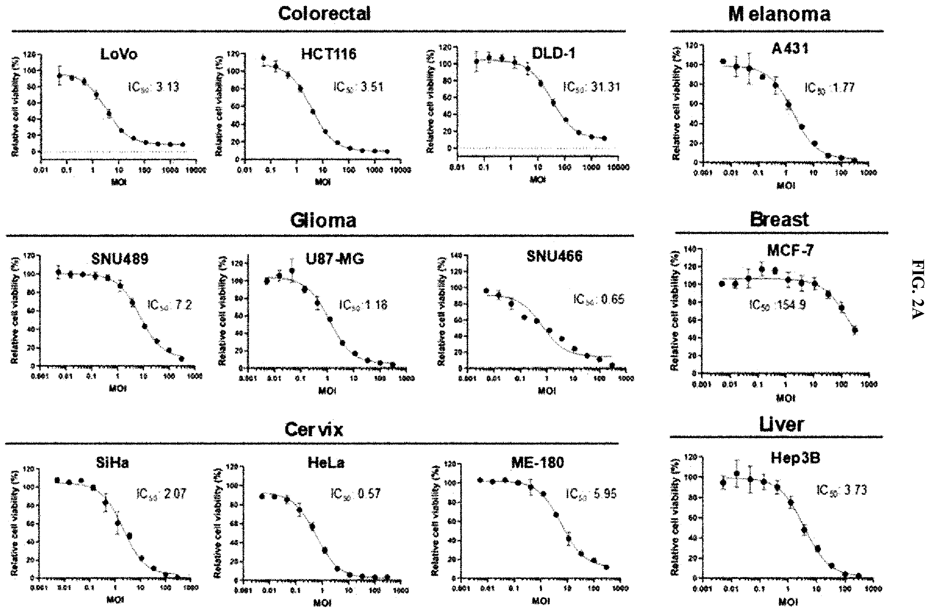

As a result, the anticancer effect of RP116 was observed in all cancer cell lines without non-responsive cell lines, and the concentration-dependent cancer cell killing effect was confirmed, verifying the dose-dependent relationship (Figure 2A).

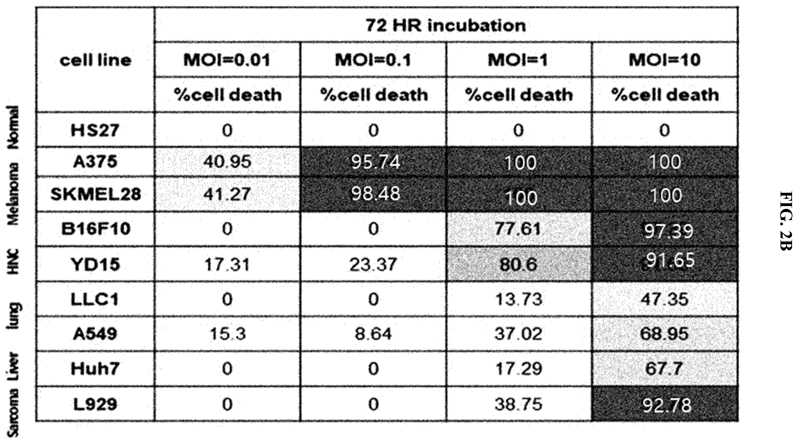

Additionally, normal cell lines (HS27), melanoma cell lines (Skmel28, A375, and B16F10), head and neck cancer cell lines (YD 15), lung cancer cell lines (LLC1, A549), liver cancer cell lines (Huh7), and stromal tumor cell lines (L929) were treated with RP116 at concentrations ranging from 0.01 to 10 MOI and incubated for 72 hours. The cell killing rate was then examined. As a result, RP116 showed no toxicity in normal cell lines but exhibited excellent anticancer effects in all cancer cells (Figure 2B).

The above experimental results demonstrate that the modified reovirus according to the present invention has anticancer effects on various cancers, including colon cancer, skin cancer, glioma, breast cancer, cervical cancer, liver cancer, and head and neck cancer.

### Example 5. Confirmation of anticancer effects of modified reovirus on skin cancer

In this example, the anticancer effects of wild-type reovirus and modified reovirus RP116 on skin cancer were compared.

Various skin cancer cell lines (B16F10, HS294T, SK-MEL-28, A375, and A431 cell lines) were either left untreated (Mock) or infected with wild-type reovirus or RP116 at various concentrations, followed by CPE (viral-induced cytopathic effects) analysis. Specifically, each cancer cell line was seeded in equal numbers in a 24-well plate, treated or untreated with the virus, and after 4 days of infection, surviving cells were stained with crystal violet to measure the survival rate. The number of surviving cells stained with crystal violet decreases as the anticancer effect of the virus increases.

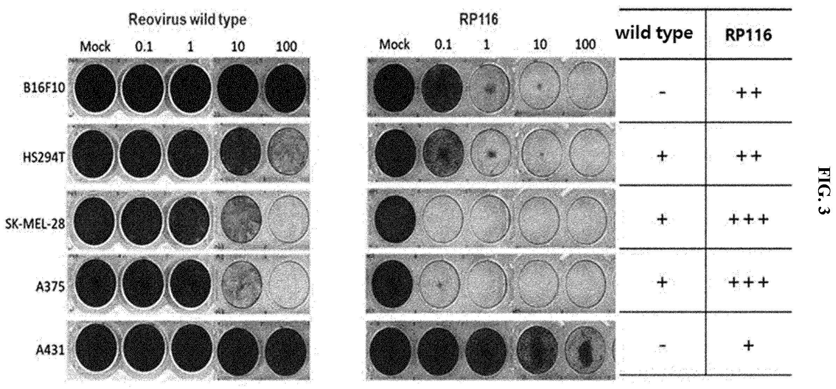

As a result, as shown in Figure 3, the survival of cancer cells decreased when some skin cancer cell lines were treated with wild-type reovirus at concentrations above 10 MOI; however, RP116 showed more pronounced cancer cell killing effects at low concentrations of 0.1 MOI in most cancer cell lines, and most cancer cells were killed at concentrations above 10 MOI. These experimental results demonstrate that the modified reovirus according to the present invention has a stronger anticancer effect on skin cancer than wild-type reovirus.

### Example 6. Confirmation of anticancer effects of modified reovirus on oral cancer

Next, the anticancer effects of wild-type reovirus and modified reovirus RP116 on oral cancer were compared.

Various oral cancer cell lines (YD-10B, YD15M, and YD 15 cell lines) were either left untreated (Mock) or infected with wild-type reovirus or RP116 at various concentrations, followed by CPE analysis. As in the previous example, each cancer cell line was seeded in equal numbers in a 24-well plate, treated or untreated with the virus, and after 4 days of infection, surviving cells were stained with crystal violet to measure the survival rate.

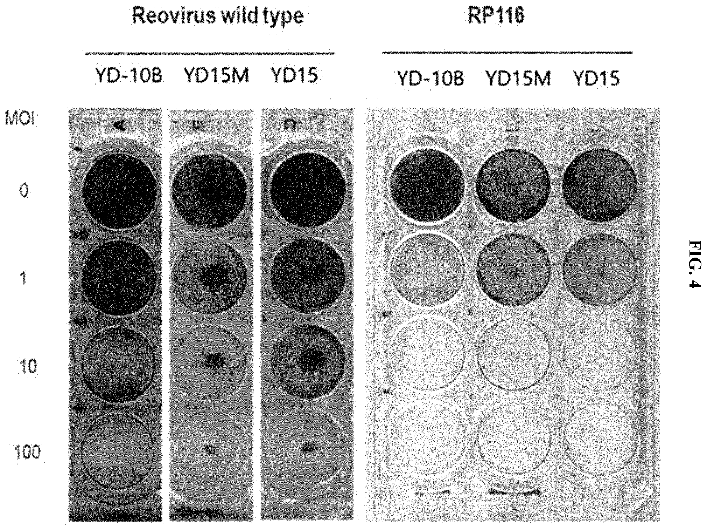

As a result, when treated with the same concentration, the survival of cancer cells was further reduced in the group treated with RP116 compared to the wild-type reovirus (Figure 4). In particular, even when treated with a high concentration of 100 MOI of wild-type reovirus, surviving cancer cells were present; however, RP116 showed almost complete cancer cell killing at a concentration of 10 MOI. These experimental results demonstrate that the modified reovirus according to the present invention also has a stronger anticancer effect on oral cancer than wild-type reovirus.

### Example 7. Verification of anticancer effects of modified reovirus on bladder cancer

In this example, the anticancer effects of wild-type reovirus and modified reovirus RP116 on bladder cancer were compared. Various bladder cancer cell lines (HT-1376, 5637, 253J, and T24 cell lines) were cultured and then seeded at a concentration of 2,000 cells/well in a 96-well plate, followed by incubation for 24 hours at 37°C and 5% CO₂. The prepared cells were treated with wild-type reovirus or RP116 at concentrations ranging from 0.1 to 10,000 MOI with 3-fold serial dilutions, and then incubated for an additional 3 days at 37°C and 5% CO₂. Cell survival was measured using a WST assay. IC₅₀ values were calculated using nonlinear regression in Prism GraphPad 9.1.0.

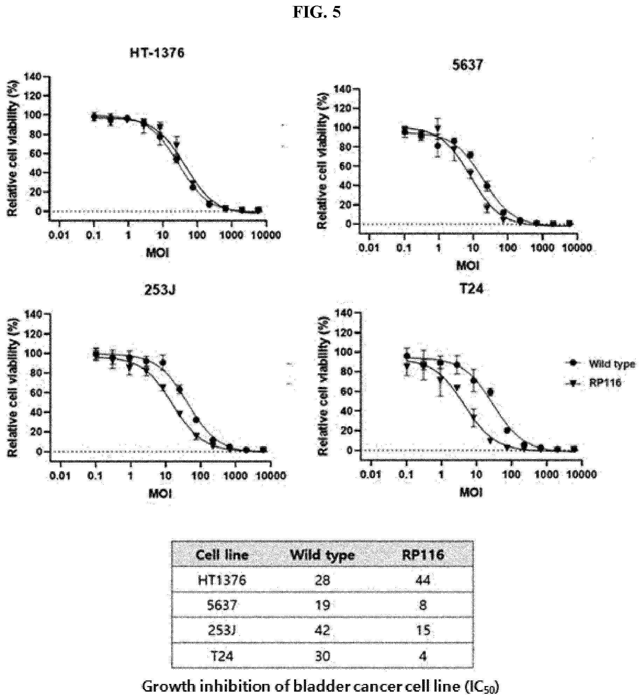

As a result, the IC₅₀ values of RP116 were lower in most bladder cancer cell lines compared to wild-type reovirus (Figure 5). These experimental results demonstrate that the modified reovirus according to the present invention has a more potent anticancer effect on bladder cancer than wild-type reovirus.

### Example 8. Verification of anticancer effects depending on administration route and dosage of modified reovirus

Having confirmed the excellent anticancer effects of RP166 on various cancer cell types, the anticancer effects of RP116 depending on the administration route and dosage were investigated using a skin cancer animal model.

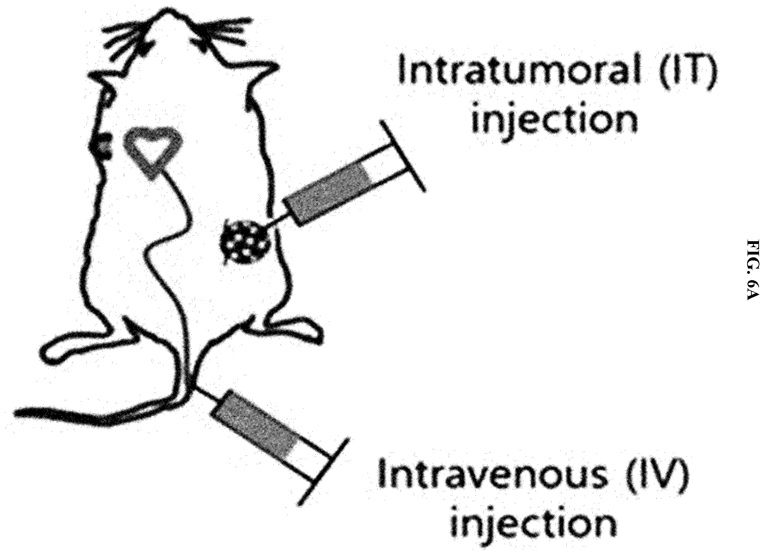

The animals used in the experiment were 6-week-old female C57BL/6 mice purchased from Nara Biotech (Seoul, South Korea). Mice were subjected to experiments after a 7-day acclimatization period in the animal laboratory, during which water and feed were not restricted. The experimental animals were provided with standardized environmental conditions, maintaining day and night at 12-hour intervals, and indoor temperature (23±2°C) was kept at an appropriate level. 1×10⁵ melanoma cell line B16F10 cells were suspended in a 1:1 ratio of 100 µL Matrigel (Corning) and phosphate-buffered saline (PBS) and subcutaneously injected into the right flank of the C57BL/6 mice. When the tumor volume reached approximately 80 mm³, RP116 was administered at doses of 1×10⁸ TCID50 or 1×10⁹ TCID50, either intratumorally (IT) or intravenously (IV) (Figure 6A). Tumor size was measured 2 to 3 times a week using calipers after virus administration, and mice were euthanized when the tumor volume exceeded 2,000 mm³. Tumor volume was calculated using the following formula: Volume = Length × Width × Width × 0.5.

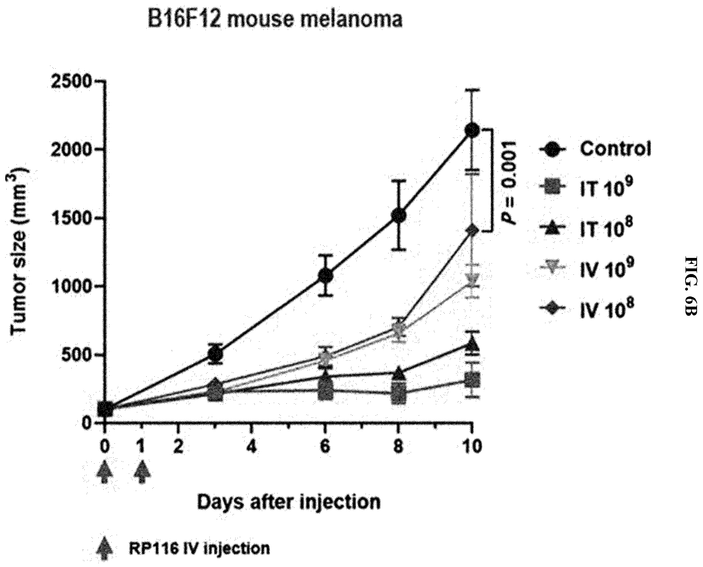

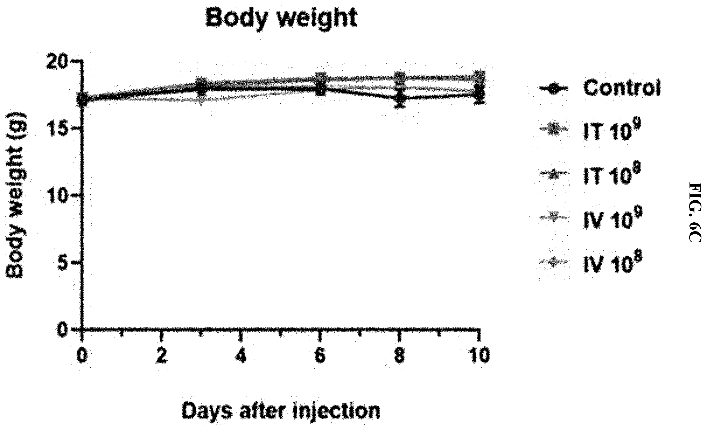

The measurement of tumor volume changes over time showed that both the IT and IV administration of RP116 in mouse models significantly inhibited tumor growth compared to the untreated control group, and a dose-dependent tumor growth inhibition effect was observed (Figure 6B). In addition, comparing the body weight of the mouse models after virus administration, there were no changes in body weight over time in both the IT and IV administered groups, and no difference compared to the untreated control group, confirming the absence of toxicity depending on the administration route or dosage (Figure 6C).

### Example 9. Verification of anticancer effects of modified reovirus based on repeated administration

Since it was confirmed in the previous examples that the modified reovirus according to the present invention exhibits excellent anticancer effects in both intratumoral and intravenous administrations, the change in the anticancer effects of the modified reovirus depending on the number of administrations was investigated.

Using the same mouse tumor model as in Example 8, when the tumor volume reached approximately 80 mm³, RP116 was administered at a dose of 1×10⁹ TCID50 for 2 times (day 0, day 1) or 5 times (day 0, day 1, day 3, day 5, day 7) intravenously (IV), and the changes in tumor volume and mouse survival rates over time were examined.

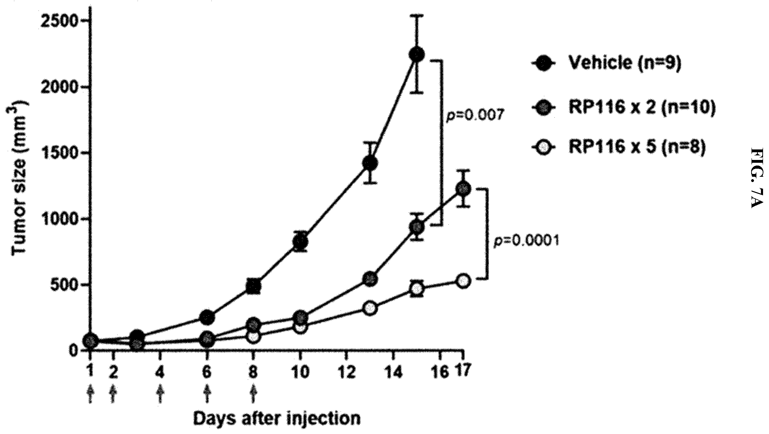

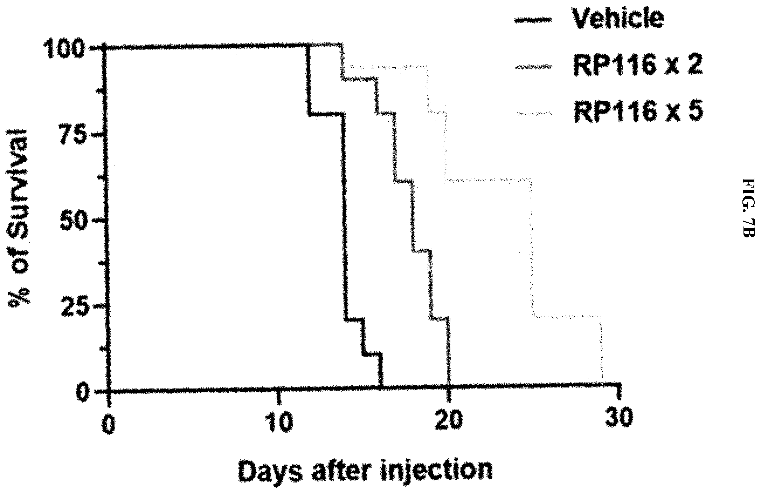

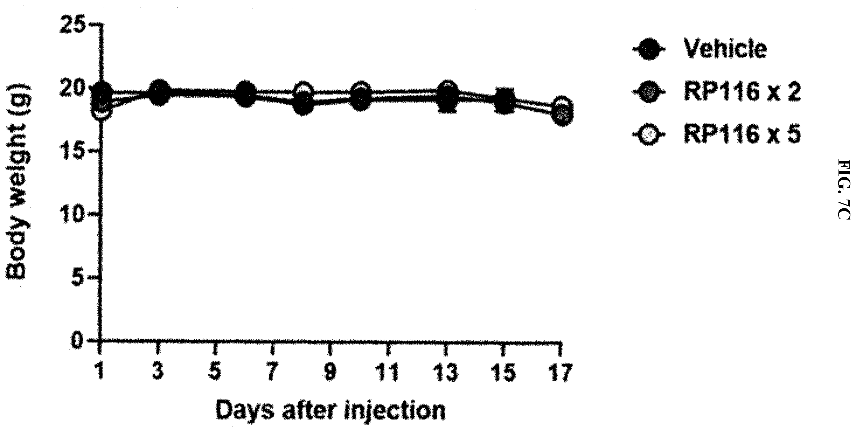

As a result, both the group receiving RP116 for 2 times and the group receiving it for 5 times significantly suppressed tumor growth and increased survival rates compared to the untreated control group. In particular, the group receiving RP116 for 5 times further delayed tumor growth and significantly improved survival rates compared to the group receiving it for 2 times (Figure 7A and 7B). The results suggest that the anticancer effects of the modified reovirus according to the present invention are further enhanced as the number of administrations increases. Meanwhile, there was no change in the body weight over time in both groups receiving RP116 for 2 or 5 times, and no difference was observed compared to the untreated control group, confirming the absence of toxicity due to repeated administration (Figure 7C).

### Example 10. Verification of the synergistic effects of modified reovirus and immune anticancer drug

Having confirmed the superior anticancer effects of the modified reovirus according to the present invention in both *in vitro* and *in vivo* experiments, the synergistic effects of the modified reovirus and an immune anticancer drug were investigated.

The experiment was divided into an untreated control group, RP116 single-administration group, and RP116 and immune anticancer drug co-administration group. A mouse tumor model similar to Example 8 was created, and when the tumor volume reached approximately 80 mm³, administration of RP116 and immune anticancer drug began. Firstly, RP116 was administered intratumorally (IT) at a dose of 1×10⁸ TCID50 for 2 times (day 0, day 1), and the co-administration group subsequently received an intraperitoneal injection of an immune checkpoint inhibitor, anti-PD-L1 antibody (Bioxcell, Cat# BE0101), at a dose of 10 mg/kg starting from day 11.

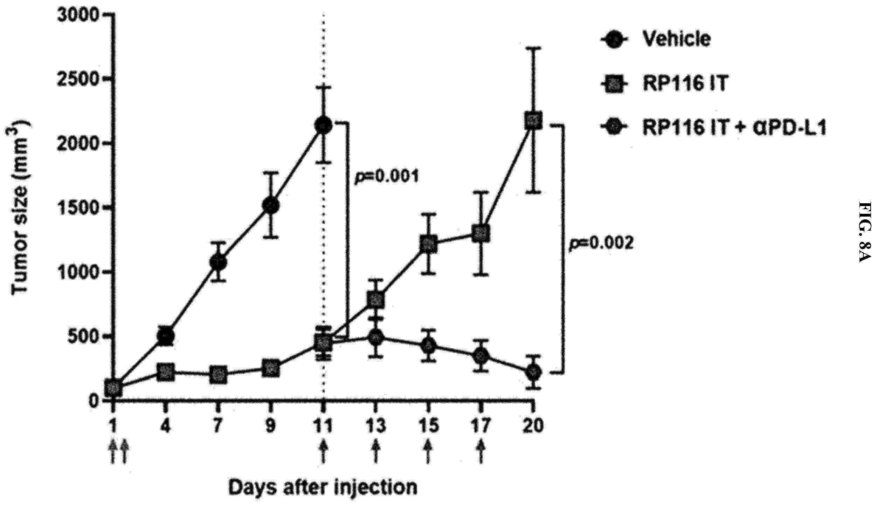

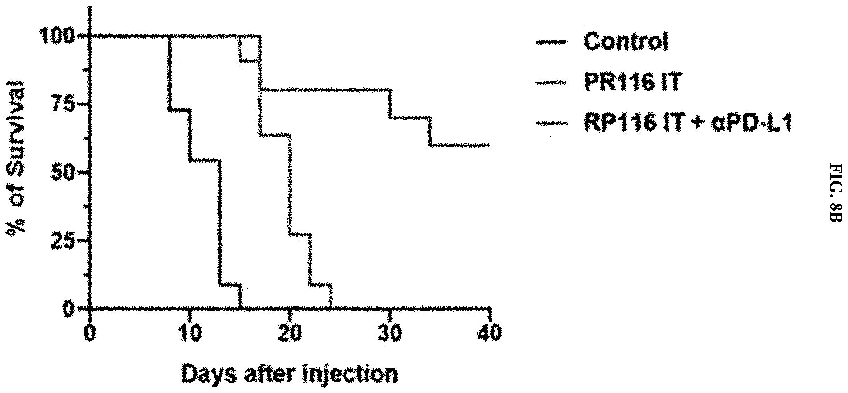

Changes in tumor volume and mouse survival rates over time were measured, and the significance was confirmed by calculating the P-value using the T-test/F-test. As a result, the untreated control group showed continuous tumor growth after the formation of the tumor in the mice. However, tumor growth was significantly suppressed by the administration of RP116, and the tumor growth was further effectively blocked by the administration of anti-PD-L1 antibody (Figure 8A). Mouse survival rates also significantly increased in the RP116 single-administration group compared to the untreated control group, and in the RP116 and anti-PD-L1 antibody co-administration group, more than half of the mice survived even after 40 days post-virus administration (Figure 8B).

### Example 11. Verification of the antitumor effect of modified reovirus in oral cancer mouse model

Having confirmed the antitumor effect of RP116 in a skin cancer mouse model in the previous examples, the antitumor effect of RP116 was further verified by creating an oral cancer mouse model.

The mouse model was prepared using a method generally similar to Example 8, except that an immunocompromised BALB/c nude mouse model was implanted with the oral cancer cell line YD10B to establish an oral cancer mouse model. When the tumor volume reached 150 mm³, RP116 was directly injected into the tumor, and changes in tumor volume over time were observed.

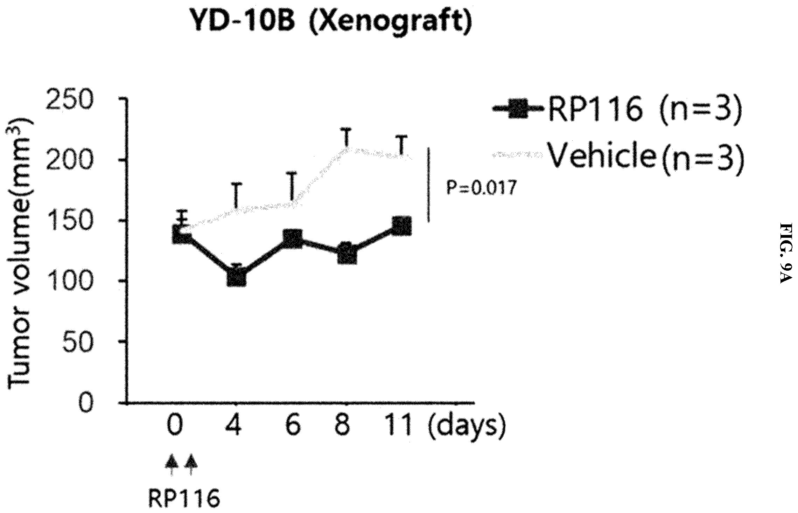

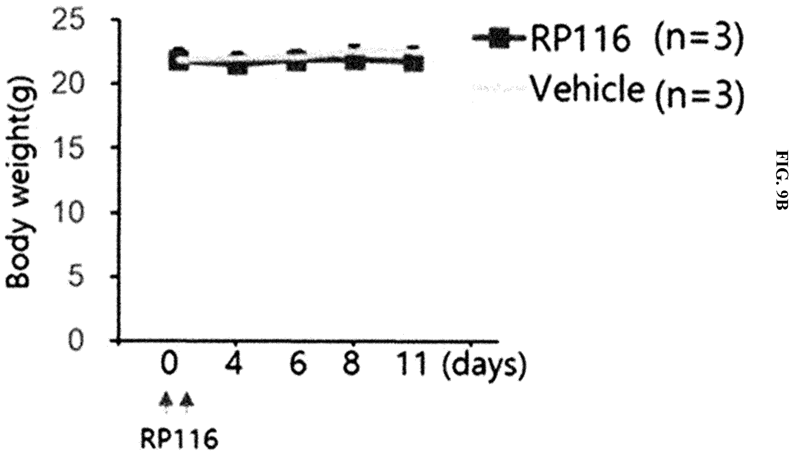

As a result, in the untreated control group, tumors continued to grow after formation in mice, whereas in the RP116-treated group, tumor growth was inhibited, and a significant reduction in tumor size was observed compared to the untreated control group (Figure 9A). Furthermore, by measuring weight changes after virus administration, no weight changes over time were observed, and no difference was found compared to the untreated control group, confirming that RP116 is non-toxic to mice (Figure 9B). These results demonstrate that the modified reovirus according to the present invention has potent antitumor effects in an oral cancer mouse model.

### Example 12. Verification of antitumor effects by cross-administration of wild-type reovirus and modified reovirus

Although oncolytic viruses can be a useful means of cancer treatment, the efficacy of oncolytic viruses may be reduced due to the formation of neutralizing antibodies during long-term intravenous administration. In this regard, the inventors noted that modified reovirus RP116 exhibited different antigenicity from wild-type reovirus, and examined whether the antitumor effect of reovirus could be enhanced by cross-administration of RP116 and wild-type reovirus.

A skin cancer mouse model was prepared as in Example 8, and when the tumor volume reached 50 mm³ or more from the implanted skin cancer cell line, viruses were intravenously administered four times (days 0, 1, 7, and 8). For accurate comparison, the group continuously treated with wild-type reovirus (WT/WT) was compared with the group treated with wild-type reovirus followed by modified reovirus (WT/RP116), and the group continuously treated with modified reovirus (RP116/RP116) was compared with the group treated with modified reovirus followed by wild-type reovirus (RP116/WT).

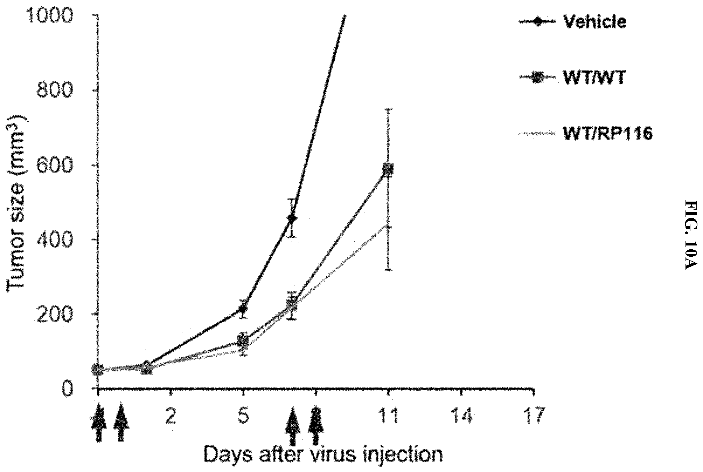

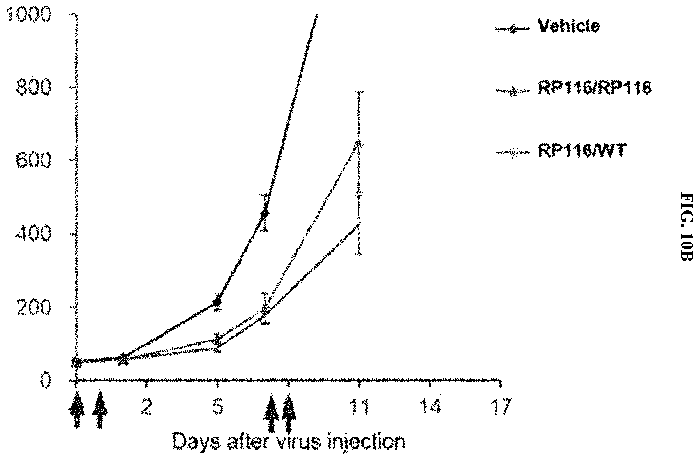

As a result, compared to the untreated control group, the WT/WT group exhibited a significant decrease in tumor growth, and the cross-administration group (WT/RP116) showed a further reduction in tumor growth (Figure 10A). Similarly, compared to the untreated control group, the RP116/RP116 group showed a significant decrease in tumor growth, and the cross-administration group (RP116/WT) demonstrated even more effective tumor growth inhibition. These results suggest that the antitumor effects of reovirus can be further enhanced by cross-treatment (RP116→WT or WT→RP116) of the modified reovirus according to the present invention and wild-type reovirus.

### Example 13. Confirmation of resistance to neutralizing antibodies of the modified reovirus

As mentioned above, neutralizing antibodies are a major cause of inhibiting the anticancer effects of anticancer viruses. Therefore, in order to verify whether the modified reovirus according to the present invention can be an effective cancer treatment, it was determined whether the modified reovirus RP116 has resistance to neutralizing antibodies induced by wild-type reovirus or RP116.

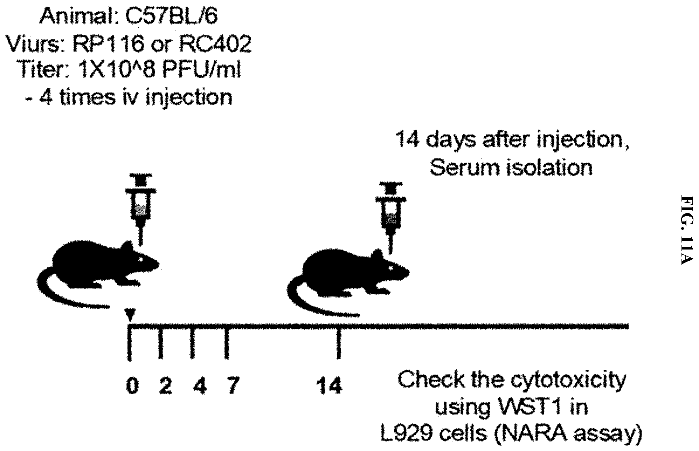

Wild-type reovirus (RC402) or modified reovirus (RP116) was intravenously administered to C57BL/6 mouse models at a dose of 1×10⁸ PFU/ml for 4 times (0, 2, 4, and 7 days), and after 14 days, serum was separated from each mouse to obtain neutralizing antibodies induced by each virus. Then, neutralizing antibodies diluted at various dilution ratios were treated along with RC402 or RP116 virus on L929 cells, and WST-1 was treated to perform cell survival analysis (Figure 11A). The experimental groups were divided as follows: RC402 virus + RC402-induced neutralizing antibody treatment group, RP116 virus + RC402-induced neutralizing antibody treatment group, RC402 virus + RP116-induced neutralizing antibody treatment group, and RP116 virus + RP116-induced neutralizing antibody treatment group.

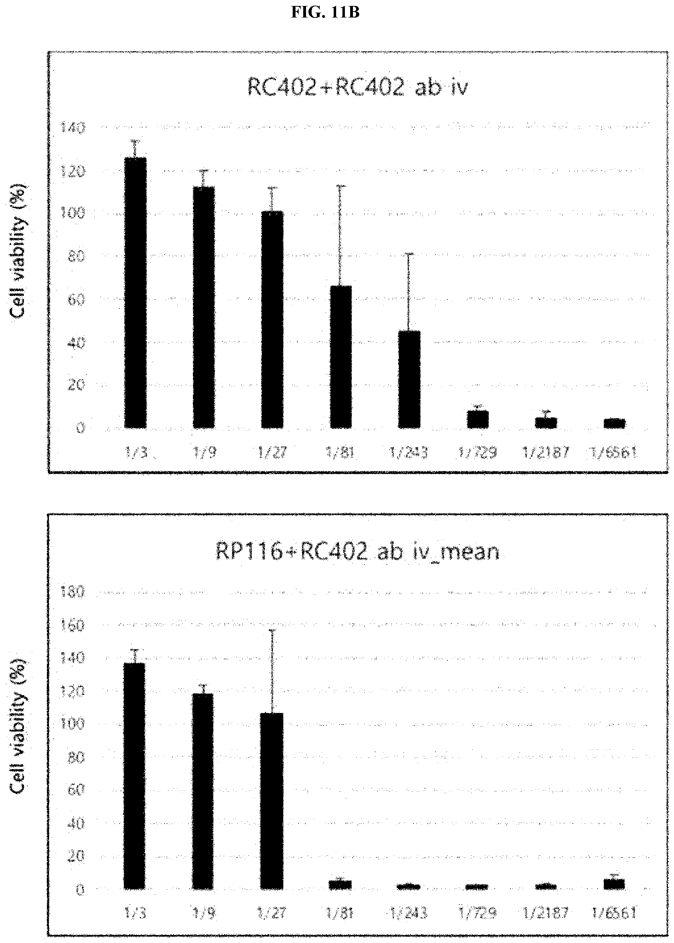

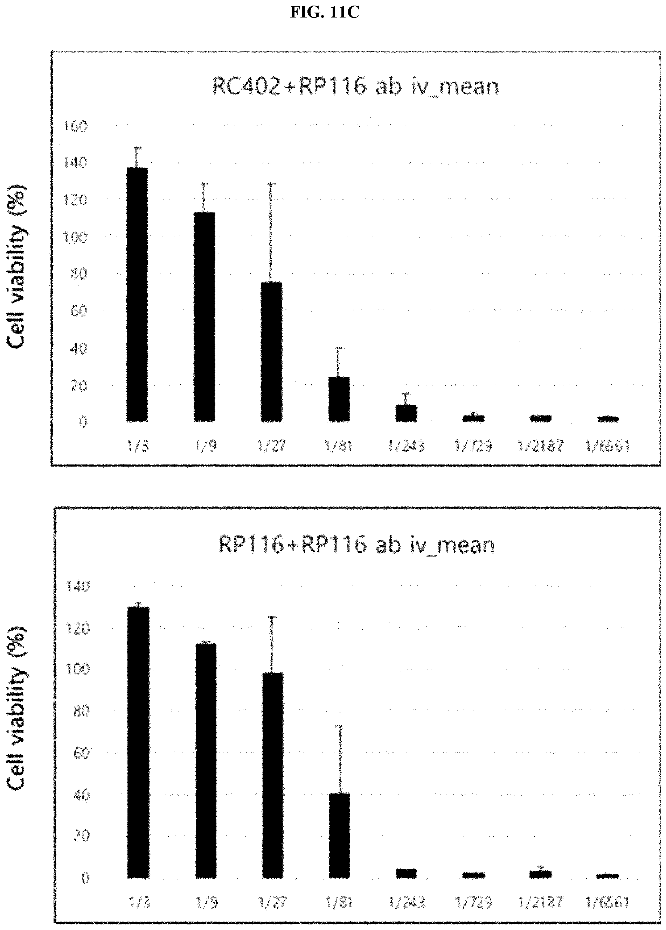

As a result, the neutralizing effect of neutralizing antibodies induced by wild-type reovirus RC402 was reduced to a negligible level when diluted up to 729 times for wild-type virus, whereas, for the modified virus RP116, the neutralizing effect completely disappeared when diluted only 81 times, indicating that the neutralizing ability against RP116 is approximately 9 times lower than that against wild-type reovirus (Figure 11B). Furthermore, RP116-induced neutralizing antibodies showed the neutralizing effect completely disappeared under similar dilution conditions of 243 times for both RC402 and RP116 (Figure 11C). The above results demonstrate that the modified reovirus according to the present invention has strong resistance to neutralizing antibodies.

As examined above, the modified reovirus according to the present invention has been confirmed to have specific anticancer effects against various cancers, including rare cancers such as melanoma and oral cancer, and the anticancer effects are superior to those of wild-type reovirus. Furthermore, the modified reovirus not only strongly suppresses tumor growth in animal models during both intratumoral and intravenous administration, allowing for a free choice of administration routes depending on the type of cancer, but also further enhances the anticancer effects through repeated administration or cross-administration with wild-type reovirus. In addition, the modified reovirus exerts more potent anticancer effects when combined with immune anticancer agents and exhibits high resistance to neutralizing antibodies, which are a drawback of anticancer viruses. Therefore, the modified reovirus according to the present invention is expected to be utilized as a new anticancer therapy and a combination drug for immune anticancer agents for treating rare cancers and the like.

The description of the present invention as mentioned above is for illustrative purposes, and a person having ordinary knowledge in the technical field to which the present invention belongs can easily understand that it can be modified into other specific forms without changing the technical concept or essential features of the present invention. Therefore, the embodiments described above should be understood as being exemplary and not limiting in all respects.

### [Industrial Applicability]

The present invention relates to a modified reovirus and its uses, and it has been confirmed that the novel modified reovirus derived from wild-type reovirus not only possesses excellent anticancer effects against various cancers, including rare cancers, but also can enhance the effects of immune anticancer agents. Specifically, the modified reovirus according to the present invention significantly reduced the survival rates of all cancer cell lines when treated with various types of cancer cells, including rare cancers such as melanoma and tongue cancer, and it was confirmed that it has excellent anticancer effects even against taxane-resistant cancer cell lines. Furthermore, the modified reovirus according to the present invention exhibited dose-dependent anticancer effects in melanoma, bladder cancer, and oral cancer mouse models, regardless of the administration route, and the anticancer effects were further increased when administered repeatedly or in cross-administration with wild-type reovirus. In particular, it was confirmed that the modified reovirus according to the present invention produces a synergistic anticancer effect when combined with immune checkpoint inhibitors. Therefore, the modified reovirus according to the present invention is expected to be usefully utilized as a new anticancer therapy and a combination drug for immune anticancer agents for treating rare cancers and the like.

## Claims

1. A modified reovirus, comprising:
a mutation in which amino acids 251 to 455 are deleted in the amino acid sequence of SEQ ID NO: 1; and
one or more mutations selected from the group consisting of a mutation in which the 963^{rd} Met in the amino acid sequence of SEQ ID NO: 2 is replaced with Val, and a mutation in which the 1265^{th} Thr in the amino acid sequence of SEQ ID NO: 2 is replaced with Ile.

2. The modified reovirus of claim 1, wherein the modified reovirus further comprises a mutation in which the 227^{th} Ile in the amino acid sequence of SEQ ID NO: 1 is replaced with Val.

3. The modified reovirus of claim 1, wherein the modified reovirus further comprises one or more mutations selected from the group consisting of:
(a) a mutation in which the 73^{rd} Glu in the amino acid sequence of SEQ ID NO: 3 is replaced with Asp;
(b) a mutation in which the 434^{th} Asp in the amino acid sequence of SEQ ID NO: 3 is replaced with Asn; and
(c) a mutation in which the 644^{th} Val in the amino acid sequence of SEQ ID NO: 3 is replaced with Ala.

4. The modified reovirus of claim 1, wherein the modified reovirus further comprises one or more mutations selected from the group consisting of
(a) a mutation in which the 64^{th} Lys in the amino acid sequence of SEQ ID NO: 4 is replaced with Glu;
(b) a mutation in which the 177^{th} Ser in the amino acid sequence of SEQ ID NO: 4 is replaced with Phe;
(c) a mutation in which the 229^{th} Glu in the amino acid sequence of SEQ ID NO: 4 is replaced with Asp; and
(d) a mutation in which the 251^{st} His in the amino acid sequence of SEQ ID NO: 4 is replaced with Leu.

5. The modified reovirus of claim 1, wherein the modified reovirus is derived from a wild-type human reovirus.

6. A pharmaceutical composition for preventing or treating cancer, comprising the modified reovirus of claim 1 as an active ingredient.

7. The pharmaceutical composition of claim 6, wherein the cancer is one or more selected from the group consisting of squamous cell carcinoma, neuroblastoma, lung cancer, lung adenoma, peritoneal cancer, skin cancer, ocular cancer, rectal cancer, perianal cancer, esophageal cancer, small intestine cancer, endocrine gland cancer, parathyroid cancer, adrenal cancer, osteosarcoma, soft tissue sarcoma, urethral cancer, blood cancer, liver cancer, gastrointestinal cancer, pancreatic cancer, choriocarcinoma, cervical cancer, ovarian cancer, bladder cancer, breast cancer, colon cancer, large intestine cancer, endometrial cancer, uterine cancer, salivary gland cancer, kidney cancer, prostate cancer, vulvar cancer, thyroid cancer, head and neck cancer, oral cancer, tongue cancer, brain cancer, and stromal tumor.

8. The pharmaceutical composition of claim 6, wherein the cancer has resistance to taxane anticancer drugs.

9. The pharmaceutical composition of claim 8, wherein the taxane anticancer drugs are one or more selected from the group consisting of paclitaxel, larotaxel, cabazitaxel, docetaxel, ortataxel, and tesetaxel.

10. The pharmaceutical composition of claim 6, wherein the pharmaceutical composition comprises the modified reovirus at a concentration of 1×10⁵ to 1×10²⁰ TCID50.

11. The pharmaceutical composition of claim 6, wherein the pharmaceutical composition is for intratumoral or intravenous administration.

12. The pharmaceutical composition of claim 6, wherein the pharmaceutical composition is administered to a subject in need thereof at least twice.

13. The pharmaceutical composition of claim 6, wherein the pharmaceutical composition is co-administered with a wild-type reovirus.

14. The pharmaceutical composition of claim 13, wherein the pharmaceutical composition is administered before or after the administration of the wild-type reovirus.

15. The pharmaceutical composition of claim 6, wherein the pharmaceutical composition further comprises an immune checkpoint inhibitor as an active ingredient.

16. The pharmaceutical composition of claim 15, wherein the immune checkpoint inhibitor is one or more selected from the group consisting of PD-1 inhibitors, PD-L1 inhibitors, PD-L2 inhibitors, OX40 inhibitors, CTLA-4 inhibitors, 4-1BB inhibitors, LAG-3 inhibitors, B7-H4 inhibitors, HVEM inhibitors, TIM4 inhibitors, GAL9 inhibitors, VISTA inhibitors, KIR inhibitors, TIGIT inhibitors, and BTLA inhibitors.

17. The pharmaceutical composition of claim 15, wherein the pharmaceutical composition is in the form of a mixture comprising the modified reovirus and the immune checkpoint inhibitor.

18. The pharmaceutical composition of claim 15, wherein the pharmaceutical composition is in the form of the modified reovirus and the immune checkpoint inhibitor each being formulated separately for simultaneous or sequential administration.

19. A kit for preventing or treating cancer, comprising a composition of any one of claims 6 to 18.

20. A pharmaceutical composition for co-administration with an immune checkpoint inhibitor, comprising the modified reovirus of claim 1 as an active ingredient.

21. The pharmaceutical composition of claim 20, wherein the pharmaceutical composition is administered simultaneously, separately, or sequentially with the immune checkpoint inhibitor.

22. A method for preventing or treating cancer, comprising administering the modified reovirus of claim 1 to a subject in need thereof.

23. Use of the modified reovirus of claim 1 for manufacturing a cancer treatment medicament.

24. Use of the modified reovirus of claim 1 for preventing or treating cancer.
